# EUROPEAN PATENT SPECIFICATION

(11) **EP 1 140 253 B1**
(45) Date of publication and mention of the grant of the patent: **09.02.2005**
(21) Application number: 00973230.6
(22) Date of filing: 02.11.2000
(51) Int. Cl.: A61M 3/02

(54) **COLONIC IRRIGATOR**
DARMIRRIGATOR
IRRIGATEUR DU COLON

(30) Priority: 02.11.1999 KR 9948109; 31.10.2000 KR 2000064404
(43) Date of publication of application: 10.10.2001
(73) Proprietor: M. I. Tech Co., Ltd., Pyungtaek-city, Kyungki-do 451-850 (KR); Kim, Jae-Hwang, Daegu-city 706-090 (KR)
(72) Inventor: KIM, Jae-Hwang, Daegu-city 706-090 (KR); AHN, Sung-Soon, Seoul 122-941 (KR); LIM, Chull-Su, Seoul 156-821 (KR)
(74) Representative: Hübner, Helmut E., Dipl.-Ing.
(86) International application number: PCT/KR2000/001247
(87) International publication number: WO 2001/032239

(56) References cited:
- EP-A2- 0 274 415
- US-A- 4 468 216
- US-A- 4 943 285
- US-A- 5 176 636
- US-A- 5 443 445

## Description

### FIELD OF THE INVENTION AND PRIOR ART

The present invention relates to a colonic irrigator, and more particularly, to a colonic irrigator that can irrigate a large intestine before performing a surgical operation with respect to an obstructive disease of a left colon, such as colon cancer, and allows the use of an endoscope so as to make it possible to identify the disease region.

Generally, two-step, three-step and one-step surgical operations are well known as methods for curing a patient of an obstruction disease of the left colon, such as colon cancer.

There are a front colon excision operation and a colonic irrigation in a one-step surgical operation. The former has problems in that a patient suffers from complications such as an obstruction of the intestine after the operation, and poor anal sphincter muscle control even after a long time has passed. The latter has problems in that a possibility of infection because of tearing is higher than with the former and cancer may be formed in the colon. The safety of a suture portion is also inferior to the rectum tissue.

Accordingly, although such a surgical operation should be performed by an expert who has much experience, and the operation time is long, it has an advantage of avoiding laparotomy. Therefore, in recent years, such a surgical operation has been widely used.

The number of patients having a disease obstructing the left intestine from cancer of the rectum and an S-shaped intestine is increased, and the rectum of the patient is generally excised.

However, such an excision of the rectum may cause complications. Therefore, to prevent the complications, it is preferable to irrigate the colon during the surgical operation.

Although colonic irrigation prevents the complications, many medical personnel and much time for the operation is required, and it is impossible to diagnose cancer formed in an intestinal tube until the surgical operation.

EP-A2-0274415 discloses a gravity fluid supplied irrigator for colon cleansing which comprises a cylindrical member to accommodate an obturator. A gravity actuated fluid supply to the irrigator is provided by a conduit leading into the cylindrical member. The conduit supply is preferably offset relative to the longitudinal axis of the cylindrical member to provide a swirling action to the entering fluid. The cylindrical member has an outwardly extending circumferential flange intermediate of the inner end and the conduit, which flange limits the insertion of the irrigator.

US-A-4468216 describes an irrigation suction catheter which comprises an elongated flexible outer tube having a proximal end connectable to a source of vacuum, a distal end having a suction inlet across the terminal tip and an aperture, near the proximal end opening to the atmosphere, but closable by the thumb or finger to close the suction circuit. Sleeved within the suction tube is an elongated flexible irrigation tube having a proximal end connectable to pressurized irrigation fluid, a distal end extending beyond the suction inlet, to form a projecting flexible tip with an irrigation discharge outlet at the terminus of the tip. A vortical circulation is set up by the irrigation outlet to guide dislodged debris into the suction inlet, when both suction and irrigation are supplied simultaneously.

US-A-4943285 discloses an undulating rectal speculum for use in conjunction with colonic lavage which has an axially undulating exterior surface for defining an annular depression to receive and be gripped by the rectal sphincter muscle upon insertion of the speculum.

US-A-5176636 dicloses a device for intubation of human passages for diagnostic and therapeutic purposes which includes a conduit surrounded by a thin walled, inflatable and longitudinally and radially expandable member which will initially drive the device into the selected passages such as the lower bowel, colon, esophagus or upper bowel. Controlled inflation of such member provides the driving, positioning force for proper intubation. Partial or total deflation of the expandable member allows sealing for therapeutic utilization, evacuation of the passage for passage inspection or combinations of such useages.

US-A-5443445 describes an intra-operative colon irrigation system which includes a drain tube having a cylindrical body with forward end formed by a dome-shaped nozzle to prevent intussusception of the bowel into the drain tube. The forward end portion of the body is inserted into the bowel, and a rear end portion has a lug with eyes securing 'cable straps' which are applied around the body and the collapsed bowel segment downstream of the bowel opening.

### PROBLEM TO BE SOLVED BY THE INVENTION

The present invention has been made in an effort to solve the above described problems of the prior art, and it is an object of the present invention to provide a colonic irrigator that can irrigate a large intestine before performing a surgical operation with respect to an obstructive disease of a left colon, such as colon cancer, and allows the use of an endoscope so as to make it possible to identify the disease region.

It is another object of the present invention to provide a colonic irrigator that can prevent an irrigating tube or an endoscope from moving when the surgical operation is performed using the irrigating tube or the endoscope.

### BRIEF DESCRIPTION OF THE DRAWINGS

FIG. 1 is a perspective view of an irrigator according to the present invention;
FIG. 2 is a side sectional view of an irrigator according to the present invention;
FIG. 3 is a view of a regurgitation preventing valve of an irrigator according to an embodiment of the present invention;
FIG. 4 is a view of a regurgitation preventing valve of an irrigator according to another embodiment of the present invention;
FIG. 5 is a side sectional view of an irrigation tube used in an irrigator according to the present invention;
FIG. 6 is a view of an example of use of an irrigator according to the present invention; and
FIG. 7 is an enlarged section view of a portion connected to an intestinal tube when an irrigator of the present invention is used; and
FIG. 8 is a view of another example of use of an irrigator according to the present invention.

### DESCRIPTION OF THE INVENTION

To achieve the above objects, the present invention provides a colonic irrigator comprising a main body for inserting into a cutting portion of an intestinal tube, the main body having a hollow portion therein, a branch tube branched off from a side of the main body and communicating with the main body, tightening means for fastening the main body on the cutting portion of the intestinal tube when the main body is inserted into the cutting portion of the intestinal tube, the tightening means being mounted on a front end of the main body, regurgitation preventing means for preventing regurgitation of contents within the intestinal tube through the branch tube when an irrigating tube or an endoscope is inserted, the colonic irrigator being characterized in that the regurgitation preventing means is mounted inside the branch tube and the tightening means comprises a fastening ring for secondary fixing an extension portion of the main body on the cutting portion (5) of the intestinal tube, the fastening ring being mounted on the front end of the main body, and a fastening nut for primary fixing an edge of the cutting portion of the intestinal tube on the extension portion, the fastening nut being spaced away from the fastening ring rearward and screw-coupled to the extension portion.

The fastening nut is ring-shaped and provided with a female thread engaged with a male thread formed on an outer circumference of the main body so as to be movable forward and rearward, the fastening nut being further provided with a depressing part depressing the edge of the cutting portion of the intestinal tube contacting the projection formed on the outer circumference of the extension portion.

In addition, the regurgitation preventing means comprises two first valves each having a half-cut line, a second valve having a central hole through which the irrigating tube or the endoscope is inserted, and a thin membrane provided on a front side of the first valves, the second valve being spaced away from the first valves.

The first valves are made of a silicone material having a predetermined elastic force, and portions of the first valves are defined in that when the irrigating tube or the endoscope selectively passes through the half-cut lines they tightly contact the outer circumference of the irrigating tube or the endoscope, thereby preventing the regurgitation of the contents of the intestinal tube.

The colonic irrigator may further comprise fixing means having a connector for fixing the first valves in the branch tube and a guide for fixing the second valve in the connector and supporting the irrigating tube or the endoscope to prevent movement.

An anti-infection tube is mounted enclosing the branch tube.

A colonic irrigator according to a preferred embodiment of the present invention will be described hereinafter with reference to the accompanying drawings.

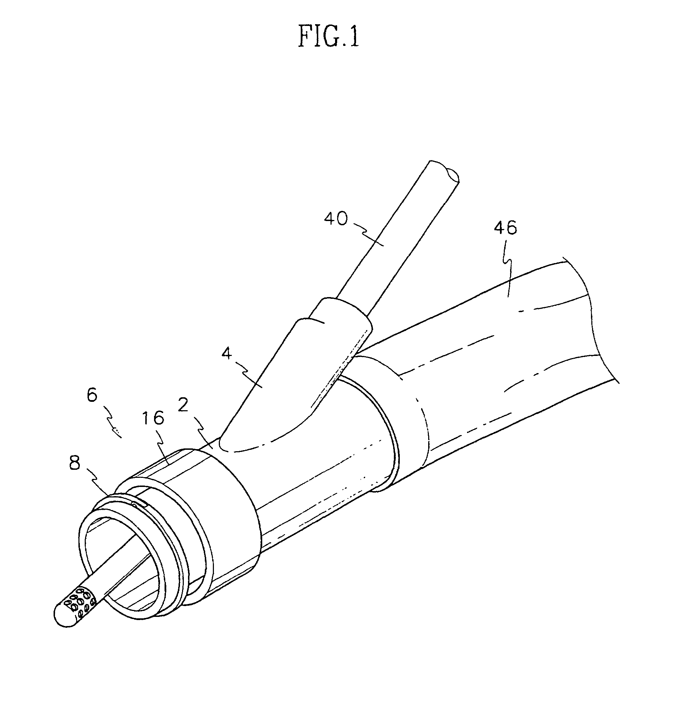

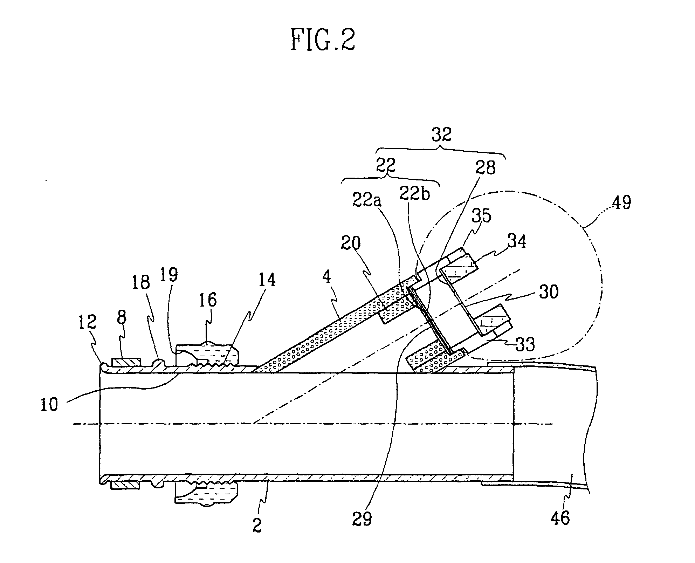

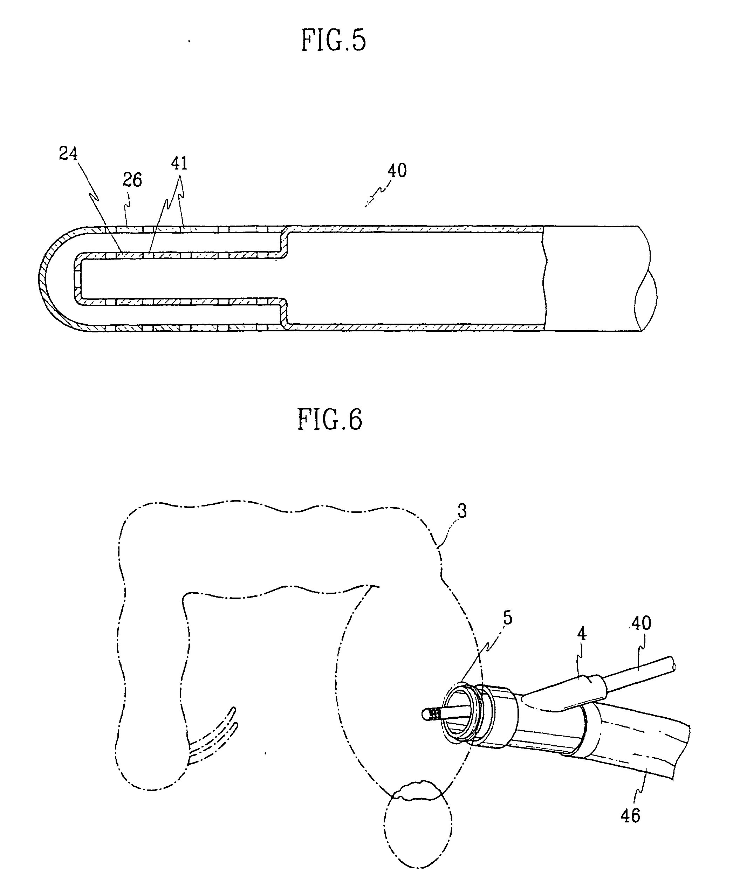

FIG. 1 is a perspective view of a colonic irrigator according to the present invention, and FIG. 2 is a side sectional view of the colonic irrigator depicted in FIG. 1. The irrigator comprises a cylindrical main body 2, a branch tube 4 extended rearward from the main body 2 at a predetermined angle, and tightening means for tightening the intestinal tube 3 (see FIG. 6) so as to prevent contents from flowing out of the intestinal tube 3, the tightening means being provided on a front end of the main body 2.

An extension portion 10 is extended from a front portion of the main body 2 and inserted into the intestinal tube 3 through a cutting portion 5 of the intestinal tube 3. A drainage tube 46 for exhausting contents and irrigation liquid out of the intestinal tube 3 is coupled to a rear portion of the main body 2.

On an outer circumference of the extension portion 10, the intestine tube 3 is fixed by a fastening ring 8. The extension portion 10 is provided with a projection 12 for preventing displacement when the intestinal tube 3 is fixed thereon.

The fastening ring 8 tightly fixes the intestine tube 3 on the outer circumference of the extension portion 10 by depressing an outer portion of the intestine tube 3 when the cutting portion 5 of the intestine tube 3 is placed on the extension portion 10.

The tightening means 6 is provided on a rear side of the fastening ring 9. The tightening means 6 comprises a male thread portion 14 formed on an outer circumference of the main body 2 and a fastening nut 16 engaged with the male thread portion 14.

The fastening nut 16 is ring-shaped and provided with a female thread. The fastening nut 16 is provided with a depressing portion 19 for depressing an outer circumference of a projection 18 formed on a border of the tightening means 6 and the extension portion 10.

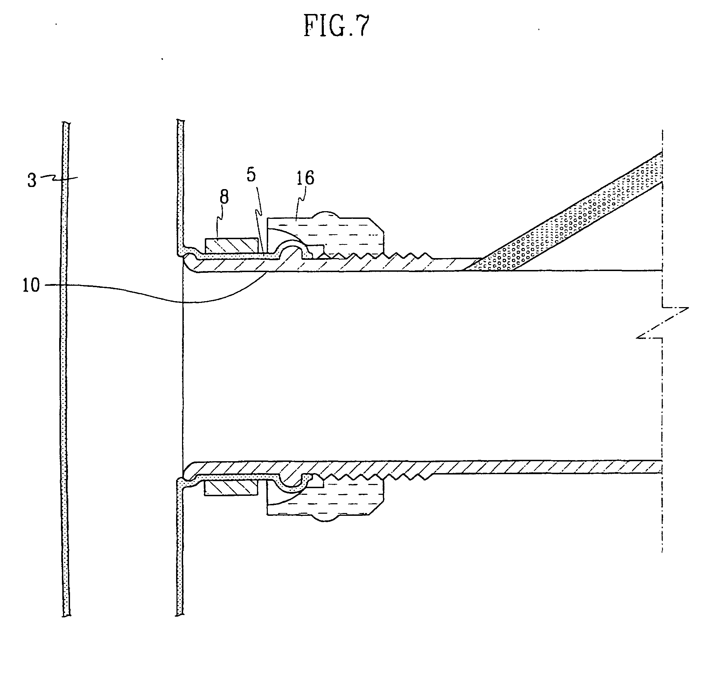

Accordingly, as shown in FIG. 7, when the fastening nut 16 is rotated, it is moved forward to depress the cutting portion 5 of the intestinal tube 3 placed on the extension portion 10.

As a result, the cutting portion 5 of the intestinal tube 3 is primarily fastened by the fastening nut 16, and secondarily depressed by the fastening ring 8.

In addition, as the branch tube 4 is connected to the main body to communicate therewith, when an irrigating tube or an endoscope 40 is inserted through the branch tube 4, the irrigating tube or the endoscope 40 can reach the intestinal tube 3 connected to the main body 2.

As described above, the branch tube 4 is inclined rearwards with respect to the main body 2 so that the irrigating tube or the endoscope 40 can be smoothly introduced into the main body. The branch tube 4 can be integrally formed with the main body 2 or can be specially made and then assembled with the main body 2.

So as to prevent the displacement of the irrigating tube or the endoscope 40 when in a state where the irrigating tube or the endoscope 40 is inserted into the branch tube 4, a flange 20 is formed extending inward of the branch tube 4.

The inward flange 20 has a diameter that can prevent the endoscope 40 or the irrigating tube from moving. A thin membrane 29 is provided on an outer side of the inward flange 20. The thin membrane 29 prevents the contents from exhausting out of the intestinal tube 3 through the branch tube before regurgitating means is mounted. The thin membrane 29 also prevents the branch tube 4 from being contaminated. The thin membrane 29 is designed to be broken when the irrigating tube or the endoscope 40 is introduced into the branch tube 4.

Regurgitating means 32 is provided at an outer side of the thin membrane 29 to prevent the contents within the main body 2 from exhausting. The regurgitating means is formed at two places.

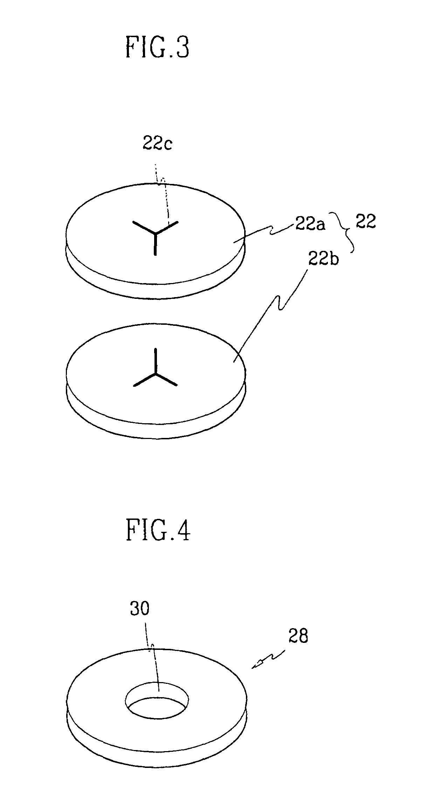

The regurgitating means is shown in FIGS. 3 and 4. That is, the regurgitating means 32 comprises first and second regurgitating members 22 and 28. As shown in FIG. 3, the first regurgitating member 22 includes two valve bodies 22a and 22b that are made of silicone material. Each center portion of the valve bodies 22a and 22b is provided with a half-cut line 22c through which the irrigating tube or the endoscope 40 is introduced.

The cut line 22c is Y-shaped, which can be broken along its shape. The valve bodies 22a and 22b are stacked on one another such that the Y-shapes of the cut lines 22c are symmetrically disposed. Accordingly, when the endoscope or the irrigating tube 40 is introduced, the broken portions of the valve bodies 22a and 22b can tightly contact the outer circumference of the endoscope or the irrigating tube 40, thereby preventing the contents from exhausting.

In addition, the second regurgitating member 28 is formed of a doughnut-shaped single valve body having a hole 30 through which the irrigating tube or the endoscope 40 can be introduced.

The first and second regurgitating members 22 and 28 are fixed on the branch tube 4 by fixing means including a connector 33 and a guide 34.

That is, the first regurgitating member 22 is connected on an end portion of the branch tube 4 by the connector 33, which is integrally coupled to the end portion of the branch tube 4 through a fitting or screw-coupling manner. At this point, the connector 33 couples the first regurgitating member 22 inside the branch tube 4 by depressing the same.

In addition, the second regurgitating member 28 is coupled to the connector by the guide 34, which is integrally coupled to the end portion of the connector through a fitting or screw-coupling manner.

An inner diameter of the guide 34 is sized such that the irrigating tube or the endoscope 40 cannot be moved by the outer force.

Furthermore, An anti-infection tube 49 is provided on an outer side of the branch tube 4. The anti-infection tube 49 prevents the irrigating tube or the endoscope 40 from being contaminated when the endoscope 40 is introduced through the branch tube 4. The anti-infection tube 49 also prevents the contents within the intestinal tube 3 from exhausting.

FIG. 6 shows a sectional view of the irrigating tube. As shown in the drawing, an end portion of the irrigating tube 40 includes a cover 24 and an inner tube 26. The cover 24 and the inner tube 26 are provided with a plurality of holes through which the irrigation liquid can be quickly injected and the liquid remained in the intestinal tube can be absorbed, if required.

A utilizing method of the colonic irrigator according to a preferable embodiment of the present invention will be described hereinafter with reference to the accompanying drawings.

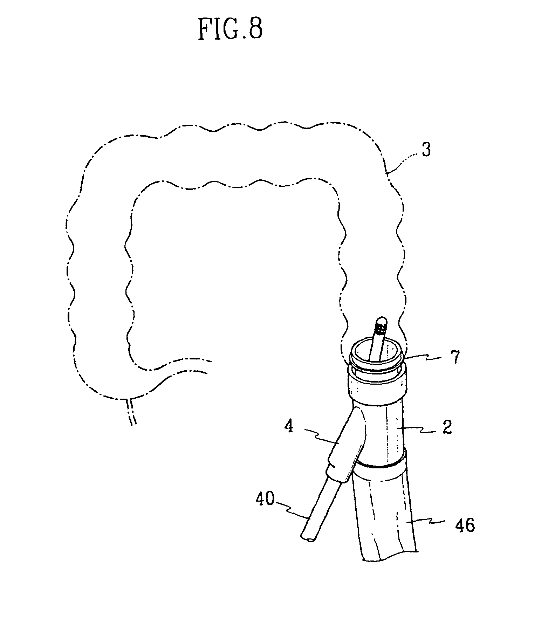

As shown in FIGS. 2 through 8, when using the irrigator, a portion of the intestinal tube 3 in which the irrigator is inserted is first selected. Then, by pressing the selected portion, the contents are moved to another portion of the intestinal tube 3, then a forceps is used to minimize the leakage of contents.

Before inserting the irrigator. the shrunken intestine tube 3 is sealed in a pouch-shape and cut in a circular-shape. Although liquid may leak during the cutting operation, the leaked liquid can be removed using an absorber.

The extension portion 10 of the main body 2 is inserted into the intestinal tube 3 through the cutting portion 5 such that the cutting portion contacts the outer circumference of the extension portion 10. In this state, The fastening nut 16 is rotated to depress the edge of the cutting portion 5 while moving forward. Then, the fastening ring 8 is fastened to fix the cutting portion 5 on the extension portion 10 of the main body 2 such that the edge of the cutting portion 5 is placed on the projection 18. In the above state, when the forceps are released, the contents within the intestine are exhausted through the main body and a drainage tube by pressing the intestinal tube 3, and then collected in a special collecting container.

While exhausting the contents, an irrigating liquid is injected through an irrigating tube 40. That is, the irrigating tube 40 is inserted into the main body through the regurgitating means 32 mounted on the branch tube 4. At this point, the irrigating tube 40 is guided by the guide 34 so that its movement can be prevented. Then, the irrigating tube 40 bursts the thin membrane 29 while passing through the hole 30 of the second regurgitating member 28 and the cutting lines 22c of the first regurgitating member 22. At this point, since the cutting portions broken along the cutting lines 22c tightly contact the outer circumference of the irrigating tube 40, leakage of the contents through the branch tube 4 can be prevented.

The irrigating liquid is continuously injected until the irrigating tube 40 reaches the appendix through the holes 41 formed on the end portion of the irrigating tube 40.

By doing the above, the inside of the intestinal tube 3 is cleansed. The amount of the irrigating liquid used for cleansing the inside of the intestinal tube 3 is about 10,000-20,000 cc. When the cleansing liquid is exhausted, the irrigating tube 40 is removed.

After the above, the endoscope 40 is inserted through the branch tube 4 to examine for other diseases such as a cancer. At this point, since the branch tube 4 is covered with the anti-infection tube 49, the branch tube 4 is not contaminated during the insertion and removal of the endoscope 40.

When it is determined that there is another disease as a result of the endoscope examination, a surgical operation is performed with respect to the examined disease after cutting the intestinal tube 3. At this point, since there are no contents within the intestine, the enlarged operation view can be obtained, making it easy to perform the operation.

In addition, as shown in FIG. 9, the colonic irrigator can be used by being inserted into not only the side of the intestinal tube 3 but also the cutting portion 7 of the intestinal tube 3.

That is, after inserting the colonic irrigator into the lower cutting portion 7 of the intestinal tube 3, the irrigating tube or the endoscope 40 is inserted into the intestinal tube 3 through the branch tube and the main body, after which the contents are exhausted through the exhausting tube 46.

### EFFECT OF THE INVENTION

As described above, the colonic irrigator according to the present invention has advantages in that it can be used to quickly operate on a patient having a chronic or acute disease with an obstructing problem of a left colon without contamination and to examine the intestinal tube using the endoscope.

In addition, when the irrigating tube or the endoscope is inserted through the branch tube of the colonic irrigator, regurgitation can be prevented by using two regurgitating members.

By mounting the guide on the branch tube, the movement of the irrigating tube or the endoscope can be prevented, improving the accuracy of the operation.

By providing the thin membrane and the anti-infection tube on the inner and outer portions of the branch tube, the contamination of the colonic irrigator can be prevented.

Although preferred embodiments of the present invention have been described in detail hereinabove, it should be clearly understood that many variations and/or modifications of the basic inventive concepts herein taught which may appear to those skilled in the present art will still fall within the spirit and scope of the present invention, as defined in the appended claims.

## Claims

1. A colonic irrigator comprising:
a main body (2) for inserting into a cutting portion (5) of an intestinal tube (3), the main body (2) having a hollow portion therein;
a branch tube (4) branched off from a side of the main body (2) and communicating with the main body (2);
tightening means (6) for fastening the main body (2) on the cutting portion (5) of the intestinal tube (3) when the main body (2) is inserted into the cutting portion (5) of the intestinal tube (3), the tightening means (6) being mounted on a front end of the main body (2); and
regurgitation preventing means (32) for preventing regurgitation of contents within the intestinal tube (3) through the branch tube (4) when an irrigating tube (40) or an endoscope (40) is inserted,
**characterized in that**
the regurgitation preventing means (32) is mounted inside the branch tube (4) and
the tightening means (6) comprises a fastening ring (8) for secondary fixing an extension portion (10) of the main body (2) on the cutting portion (5) of the intestinal tube (3), the fastening ring (8) being mounted on the front end of the main body (2), and a fastening nut (16) for primary fixing an edge of the cutting portion (5) of the intestinal tube (3) on the extension portion (10), the fastening nut (16) being spaced away from the fastening ring (8) rearward and screw-coupled to the extension portion (10).

2. A colonic irrigator of claim 1 wherein the fastening nut (16) is ring-shaped and provided with a female thread engaged with a male thread formed on an outer circumference of the main body (2) so as to be movable forward and rearward, the fastening nut (16) being further provided with a depressing part (19) depressing the edge of the cutting portion (5) of the intestinal tube (3) contacting the projection (18) formed on the outer circumference of the extension portion (10).

3. A colonic irrigator of claim 1 wherein the regurgitation preventing means (32) comprises two first valves (22a, 22b) each having a half-cut line (22c), a second valve (28) having a central hole (30) through which the irrigating tube (40) or the endoscope (40) is inserted, and a thin membrane (29) provided on a front side of the first valves (22a, 22b), the second valve (28) being spaced away from the first valve (22a, 22b).

4. A colonic irrigator of claim 1 wherein each of the first valves (22a, 22b) is made of a silicone material having a predetermined elastic force, portions of the first valves (22a, 22b) defined in that when the irrigating tube (40) or the endoscope (40) selectively passes through the half-cut lines (22c) they tightly contact the outer circumference of the irrigating tube (40) or the endoscope (40), thereby preventing the regurgitation of the contents with the intestinal tube (3).

5. A colonic irrigator of claim 4 further comprising fixing means having a connector (33) for fixing the first valves (22a, 22b) in the branch tube (4) and a guide (34) for fixing the second valve (28) in the connector (33) and supporting the irrigating tube (40) or the endoscope (40) to prevent the movement.

6. A colonic irrigator of claim 1 wherein an anti-infection tube (49) is mounted enclosing the branch tube (4).

## Patentansprüche

1. Darmirrigator mit
einem Hauptkörper (2) zum Einführen in einen Schnittbereich (5) einer Darmröhre (3), wobei der Hauptkörper (2) einen hohlen Bereich aufweist;
eine Abzweigröhre (4), die von einer Seite des Hauptkörpers (2) abgezweigt ist und mit dem Hauptkörper (2) in Verbindung steht;
einer Festzieheinrichtung (6) zur Befestigung des Hauptkörpers (2) am Schnittbereich (5) der Darmröhre (3), wenn der Hauptkörper (2) in den Schnittbereich (5) der Darmröhre (3) eingeführt wird, wobei die Festzieheinrichtung (6) auf einem vorderen Ende des Hauptkörpers (2) angebracht ist; und
einer Regurgitationsverhinderungseinrichtung (32) zur Verhinderung der Regurgitation des Inhalts der Darmröhre (3) durch die Abzweigröhre (4), wenn eine Irrigationsröhre (40) oder ein Endoskop (40) eingeführt wird,
**dadurch gekennzeichnet, dass**
die Regurgitationsverhinderungseinrichtung (32) innerhalb der Ab.zweigröhre (4) angeordnet ist und
die Festzieheinrichtung (6) einen Befestigungsring (8) zur sekundären Befestigung eines Verlängerungsabschnitts (10) des Hauptkörpers (2) auf dem Schnittbereich (5) der Darmröhre (3) umfasst, wobei der Befestigungsring (8) auf dem vorderen Ende des Hauptkörpers (2) angeordnet ist, und eine Befestigungsmutter (16) zur primären Befestigung des Rands des Schnittbereichs (5) der Darmröhre (3) auf dem Verlängerungsabschnitt (10), wobei sich die Befestigungsmutter (16) in einem Abstand hinter dem Befestigungsring (8) befindet und mit dem Verlängerungsabschnitt (10) über ein Gewinde verbunden ist.

2. Darmirrigator nach Anspruch 1, wobei die Befestigungsmutter (16) ringförmig ist und ein Innengewinde aufweist, das mit einem an einem äußeren Umfang des Hauptkörpers (2) ausgebildeten Außengewinde in Eingriff ist, um nach vorne und nach hinten bewegbar zu sein, wobei die Befestigungsmutter (16) ferner mit einem Druckabschnitt (19) versehen ist, der den Rand des Schnittbereichs (5) der Darmröhre (3) hinunterdrückt, der an dem am äußeren Umfang des Verlängerungsabschnitts (10) gebildeten Vorsprung (18) anliegt.

3. Darmirrigator nach Anspruch 1, wobei die Regurgitationsverhinderungseinrichtung (32) zwei erste Ventile (22a, 22b) mit jeweils einer Halbschnittlinie (22c), ein zweites Ventil (28) mit einem mittigen Loch (30), durch das die Irrigationsröhre (40) oder das Endoskop (40) eingeführt wird, und eine dünne Membran (29) umfasst, die auf der Vorderseite der ersten Ventile (22a, 22b) vorgesehen ist, wobei das zweite Ventil (28) vom ersten Ventil (22a, 22b) beabstandet ist.

4. Darmirrigator nach Anspruch 1, wobei jedes der ersten Ventile (22a, 22b) aus einem Silikonmaterial von bestimmter Elastizität hergestellt ist, wobei Bereiche der ersten Ventile (22a, 22b) dadurch definiert sind, dass sie, wenn wahlweise die Irrigationsröhre (40) oder das Endoskop (40) durch die Halbschnittlinien (22c) hindurchtritt, den äußeren Umfang der Irrigationsröhre (40) oder des Endoskops (40) eng umschließen und so die Regurgitation des Inhalts der Darmröhre (3) verhindern.

5. Darmirrigator nach Anspruch 4, der desweiteren Befestigungsmittel mit einem Verbindungsstück (33) zum Fixieren der ersten Ventile (22a, 22b) in der Abzweigröhre (4) und eine Führung (34) zum Fixieren des zweiten Ventils (28) im Verbindungsstück (33) und zum Stützen der Irrigationsröhre (40) oder des Endoskops (40) aufweist, um Bewegungen zu verhindern.

6. Darmirrigator nach Anspruch 1, wobei eine Anti-Infektions-Röhre (49) angeordnet ist, die die Abzweigröhre (4) umgibt.

## Revendications

1. Irrigateur du colon comprenant :
un corps principal (2) à insérer dans une portion de découpage (5) d'un conduit intestinal (3), le corps principal (2) ayant une portion creuse dans celui-ci ;
un tube secondaire (4) bifurquant à partir d'un côté, du corps principal (2) et communiquant avec le corps principal (2) ;
des moyens de serrage (6) pour attacher le corps principal (2) sur la portion de découpage (5) du conduit intestinal (3) quand le corps principal (2) est inséré dans la portion de découpage (5) du conduit intestinal (3), les moyens de serrage (6) étant monté sur une extrémité avant du corps principal (2) ; et
des moyens de prévention du dégorgement (32) pour prévenir le dégorgement du contenu de l'intérieur du conduit intestinal (3) par le tube secondaire (4) quand on insère un tube d'irrigation (40) ou un endoscope (40),
**caractérisé en ce que**
les moyens de prévention du dégorgement (32) sont montés à l'intérieur du tube secondaire (4) et
les moyens de serrage (6) comprennent une bague de fixation (8) pour une fixation secondaire d'une portion d'extension (10) du corps principal (2) sur la portion de découpage (5) du conduit intestinal (3), la bague de serrage (8) étant montée sur l'extrémité avant du corps principal (2), et un écrou de fixation (16) pour une fixation primaire d'un bord de la portion de découpage (5) du conduit intestinal (3) sur la portion d'extension (10), l'écrou de fixation (16) étant espacé de la bague de fixation (8) vers l'arrière et couplé par vissage à la portion d'extension (10).

2. Irrigateur du colon selon la revendication 1, dans lequel l'écrou de fixation (16) est en forme de bague et présente un filetage femelle engagé avec un filetage mâle formé sur une circonférence extérieure du corps principal (2) de manière à pouvoir être déplacé vers l'avant et vers l'arrière, l'écrou de fixation (16) présentant en outre une partie abaissante (19) abaissant le bord de la portion de découpage (5) du conduit intestinal (3) en contact avec la saillie (18) formée sur la circonférence extérieure de la portion d'extension (10).

3. Irrigateur du colon selon la revendication 1, dans lequel les moyens de prévention du dégorgement (32) comprennent deux premiers clapets (22a, 22b) ayant chacun une ligne semi-découpée (22c), un second clapet (18) ayant un trou central (30) à travers lequel est inséré le tube d'irrigation (40) ou l'endoscope (40), et une membrane fine (29) fournie sur un côté avant des premiers clapets (22a, 22b), le second clapet (28) étant espacé du premier clapet (22a, 22b).

4. Irrigateur du colon selon la revendication 1, dans lequel chacun des premiers clapets (22a, 22b) est fabriqué dans un matériau de silicone ayant une force élastique prédéterminée, des portions de premiers clapets (22a, 22b) définies en ce que quand le tube d'irrigation (40) ou l'endoscope (40) passe de manière sélective à travers les lignes semi-découpées (22c), ils sont en contact étroit avec la circonférence extérieure du tube d'irrigation (40) ou de l'endoscope (40), empêchant de cette manière le dégorgement du contenu dans le conduit intestinal (3).

5. Irrigateur du colon selon la revendication 4, comprenant en outre des moyens de fixation ayant un connecteur (33) pour fixer les premiers clapets (22a, 22b) dans le tube secondaire (4) et un guide (34) pour fixer le second clapet (28) dans le connecteur (33) et maintenir le tube d'irrigation (40) ou l'endoscope (40) pour bloquer le mouvement.

6. Irrigateur du colon selon la revendication 1, dans lequel un tube anti-infection (49) est monté enveloppant le tube secondaire (4).
